# EUROPEAN PATENT SPECIFICATION

(11) **EP 4 042 989 B1**
(45) Date of publication and mention of the grant of the patent: **09.07.2025**
(21) Application number: 22156411.5
(22) Date of filing: 11.02.2022
(51) Int. Cl.: A61F 7/02, A61F 7/10, A61K 8/02, A61K 8/34

(54) **LIQUID COMPOSITION FOR COLD APPLICATOR SET**
FLÜSSIGE ZUSAMMENSTELLUNG FÜR EIN KÜHLAPPLIKATORSET
COMPOSITION LIQUIDE POUR UN ENSEMBLE APPLICATEUR DE FROID

(30) Priority: 11.02.2021 ES 202130277 U
(43) Date of publication of application: 17.08.2022
(73) Proprietor: Lidermed, S.A., 08302 Mataró (ES)
(72) Inventor: MONTERO ALFONSO, Manuel, 08302 Mataró (ES)
(74) Representative: Manresa Medina, José Manuel

(56) References cited:
- EP-A1- 2 716 266
- WO-A1-2006/038965
- WO-A1-2015/061829
- WO-A1-2018/056579
- WO-A2-2007/037707
- ES-U- 1 226 290
- US-A1- 2006 293 736
- US-A1- 2015 201 624

## Description

The present invention concerns a cold applicator assembly, wherein a tubular fabric immersed in a liquid composition is contained in an airtight pack, wherein said liquid composition comprises: water or distilled water, about 50-60% of the total, alcohol denat, about 20-28% of the total, an anti-freeze agent, about 15-20% of the total, a detergent, about 1,5-2,5% of the total, a vasodilator product 0,2-0,7% of the total, relaxant products 0,7-1% of the total, and preservatives in a proportion of about 0,075-0,090% of the total.

### BACKGROUND OF THE INVENTION

Different patents and utility models are known in the state of the art that refer to sealed packs or containers that are transformed into cold packs.

Thus, known in the art is the Spanish Utility Model n° 0280067, "INSTANT HOT OR COLD PACK FOR TRANSFERING HEAT TO OR FROM AN OBJECT", of the year 1984, now in the public domain, in the name MINNESOTA MINING AND MANUFACTURING COMPANY, which refers to an instant hot or cold pack to transfer heat to or from an object, intended to be reusable as a cold pack, which comprises: a) a first compartment containing a predetermined amount of a solvent, comprised primarily of water; b) a second compartment containing a predetermined amount of a particulate solute capable of essentially completely dissolving in the solvent, which releases or absorbs a predetermined amount of heat; c) means for blending the contents of the first compartment and the second compartment at the time of use, so that the solute can dissolve in the solvent; d) a third compartment containing a predetermined amount of a gelling agent capable of gelling with the solvent solution and solute at room temperature and producing a gel that is relatively soft and moldable when cooled at temperatures at least as low as -18°C; and means for blending the contents of the third compartment with the previously mixed contents of the first compartment and the second compartment, so that the gelling agent can be gelled with the solvent solution and solute. It belongs to the state of the art, the Spanish Utility Model n° 201531207 (ES1147037) "COOLING RECOVERY MESH", of the year 2015, in the name of Francisco Luis PEREZ HUMANES, which refers to a cooling recovering mesh, characterized in that it can be carried out as an item of clothing that reaches the knees or the ankles, intended to be used tight to the body and which additionally contains inside one or more bag-like compartments containing a non-toxic and bio-degrading viscous gel capable of resisting low temperatures without solidifying, internally lined by a layer or lining made of adequate fabric which avoids burning the skin when in contact with the cold.

This same owner company has registered Utility Model n° 201930115 (ES1226290) "COLD APPLICATOR ASSEMBLY", of the year 2019, which refers to a cold applicator assembly of the type comprising a tubular fabric made of at least cotton and elastomer, characterized in that it comprises an airtight pack inside which the tubular fabric and the liquid composition that covers the tubular fabric are arranged; said liquid composition comprising water, alcohol and at least one analgesic and/or anti-inflammatory product; the tubular fabric comprising the following specifications: cotton about 70-94% of the total, elastomer, about 3-15% of the total, and polyester, about 3-15% of the total, and said liquid composition comprising: water or distilled water, about 5055% of the total, alcohol, about 42-47% of the total, and an analgesic and/or anti-inflammatory product, about 1-5% of the total.

WO2018056579A1 discloses an apparatus for cooling a body part, the apparatus including a cooling composition, a bandage and a tubular pad.

### BRIEF DESCRIPTION OF THE INVENTION

This application falls within the field of assemblies that immediately apply cold to the skin by different means.

One prior art document is Utility Model n° ES1226290, owned by the same applicant company.

In said utility model indicates that the problem that the scope of cooling is determined by the area covered by the gel is solved so that in a limb there would be many parts that could not cooled at once. The solution offered is an applicator assembly that allows that the parts covered by the tubular fabric are cooled simultaneously, that is, if a person wears a sock or a sleeve according to the invention, the whole limb will be cooled at once.

In addition, it is advantageous in that, while the limb is being cooled, analgesic and/or anti-inflammatory products can be applied thereto that aid to the recovery of said limb.

Even when that invention is effective and fully valid, there are certain problems in some establishments and due to the regulations of some countries as regards the volume of alcohol contained in the liquid composition.

For this reason, it has become necessary to develop a new liquid composition that allows the product to be sold to any type of institution.

Thus, the alcohol content, which was preferably ethyl alcohol, has been reduced to approximately half, replacing it by alcohol denat, and allowing the product not to be considered a flammable product. Besides, by reducing the ethyl alcohol contents to almost half, its drying effect on the skin is avoided.

An anti-freeze agent is added to prevent the cold from damaging the skin, since the product can be supplied in different locations (such as athletic tracks, ski tracks, etc.) and at different outside temperatures, additionally to the exogenous reaction that the alcohol when contacting the skin, which could cause the skin temperature to drop too low and burn the skin. Hence, the amount of anti-freeze agent is quite similar to that of alcohol denat.

To allow several reuses thereof, it has been planned to add a preservative to prevent the composition from degrading.

An object of the present invention is a liquid composition for cold applicator assembly, wherein a tubular fabric contained in a pack is immersed in such liquid, characterized in that said liquid comprises: water or distilled water, about 50-60% of the total, alcohol denat, about 20-28% of the total, an anti-freeze agent, about 15-20% of the total, a detergent, about 1,5-2,5% of the total, a vasodilator product 0,2-0,7% of the total, relaxant products 0,7-1% of the total, and preservatives in a proportion of about 0,075-0,090% of the total.

### BRIEF DESCRIPTION OF THE DRAWINGS

To facilitate the explanation hereof, this specification includes two sheets of drawings representing a practical embodiment, which is cited by way of example only and shall not limit the scope of the present invention:
- Figure 1 is a view of the application of the liquid in a cold applicator assembly, and
- Figure 2 is a view of the application of the tubular fabric, such as socks, in the lower limbs.

### PRACTICAL EMBODIMENT OF THE PRESENT INVENTION

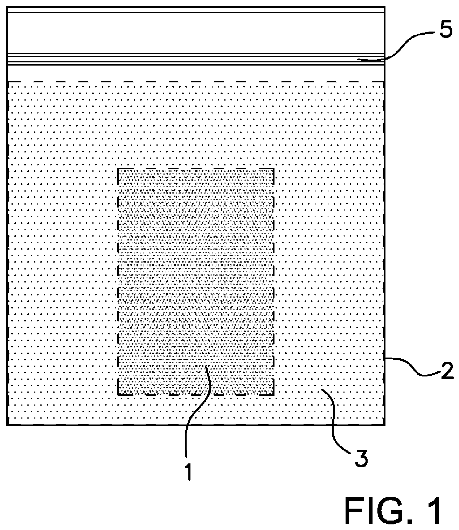

Thus, Figure 1 illustrates a pack 2 with closing 5, a tubular fabric 1 and a liquid composition 3.

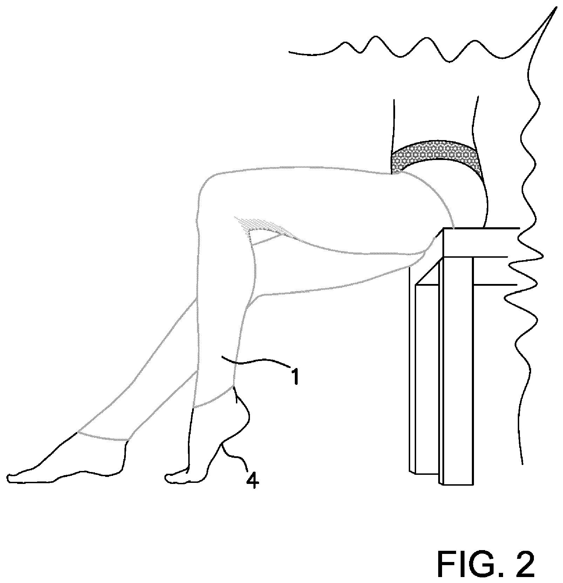

Figure 2 represents the tubular fabric 1 and User 4

In a practical embodiment, the cold applicator assembly comprises a tubular fabric 1; in this embodiment it is, as shown in Figure 2, socks.

Said tubular fabric 1 is made of cotton, polyester and elastomer. The elastomer yarn (for example, latex) is covered with polyester (for example, two layers), as indicated in the above-cited Utility Model ES1226290, owned by the same applicant company.

The assembly comprises an airtight pack 2 (Figure 1), inside which the tubular fabric 1 and a liquid composition 3 are arranged, so that the tubular fabric 1 becomes immersed in or covered by the liquid composition 3.

Basically, the liquid composition 3 comprises water or distilled water, alcohol denat, an anti-freeze agent, detergent, a vasodilator product, relaxant products, and preservatives.

Once the use of the tubular fabric 1 is finished, it can be reused by reintroducing it into the pack 2, immersing it in the liquid composition 3 and closing the pack 2 with the upper closing 5. The preservative agent, as well as the disinfectant, antiseptic, antibacterial and antimicrobial products help to preserve the liquid and allow to reuse it, as will be explained bellow, in more detail.

As this is a product that can be purchased in sports-goods shops, supermarkets, etc., in view of the restrictions in force in certain Countries and the reluctance of some users, the inventors have conceived the use of alcohol denat, which prevents the intake of said alcohol and, at the same time, is less flammable than ethanol alcohol.

To overcome the reluctances of certain jurisdictions, which may consider this a flammable product, and that the product can be sold without restrictions in almost all types of stores and institutions, a reduction of a percentage of alcohol has been envisaged to reduce the flammability of the product, which has been reduced up to 20% of the total by using alcohol denat.

In addition, water is used, preferably distilled water, since the properties thereof are, in principle, unchanging everywhere.

Moreover, to avoid the degradation of the liquid composition, the possibility of adding an anti-freeze agent, such as propylene glycol, has been considered. This facilitates the task of preventing the formation of ice crystals that might burn the user's skin.

The relaxant products can be menthol and camphor. The relaxing properties of these elements, including relieving and soothing discomfort, are especially useful for small injuries that an athlete may undergo, after of exertion.

Finally, to increase the durability of the liquid composition, preservatives can be added, allowing the product to be used several times.

Optionally, disinfectants, antiseptics and antimicrobials can also be added to both increase the preservation of the liquid composition (by eliminating the bacterial and microbial agents that may damage the solution) and to help the users with a first treatment, in the case of chafing (antiseptics and disinfectants).

The liquid composition proportions could be as follows:

| Liquid composition | Proportion in the total composition |
|---|---|
| Water or distilled water | 50-60% |
| Alcohol denat | 20-28% |
| Anti-freeze agent | 15-20% |
| Relaxant product | 0,7-1% |
| Preservatives | 0,075-0,090% |
| Detergents | 1,5-2,5% |
| Vasodilator product | 0,2-0,7% |

If disinfectant, antibacterial, antiseptic, and antimicrobial products were added, the proportion would be about 0,2-0,4% of the total.

Propylene glycol could be used as anti-freeze agent, as it is considered harmless to human health.

Tri-ethylene glycol could be used as disinfectant, for it is considered a green product, that is, harmless for the environment plus miscible with water and with a large number of components, such as glycerin.

A possible vasodilator could be methyl salicylate, which also has relaxing properties and generates a heating effect in the area when the liquid composition is removed.

Caprylyl glycol can be used as a preservative, which is also used in cosmetics as a skin conditioner.

A detergent with moisturizing properties such as polysorbate 20 will also be used, due to its non-toxicity.

Glycerin will be used as an antibacterial product, which also has moisturizing properties for the skin.

Cetylpyridinium chloride will be used as antiseptic. Typically used for oral hygiene, in this case, it is used for its low activity against the skin.

Methyl lauroyl arginate HCL is an antimicrobial with preservative properties.

The proportions will vary depending on the effect sought: for a more cooling effect, increasing the menthol; for a more heating effect, increase the vasodilator, and so on. The compositions should be present within the indicated ranges, even when they may vary according to the brands and the manufacturers used.

The present invention describes a new cold applicator assembly. The examples mentioned herein are not limitative of the present invention, which may have different applications and/or adaptations, all of which within the scope of the following claims.

## Claims

1. Cold applicator assembly, wherein a tubular fabric (1) immersed in liquid composition is contained in an airtight pack, wherein said liquid composition comprises:
- Water or distilled water, about 50-60% of the total,
- Alcohol denat, about 20-28% of the total,
- An anti-freeze agent, about 15-20% of the total,
- A detergent, about 1,5-2,5% of the total,
- A vasodilator product 0,2-0,7% of the total,
- Relaxant products 0,7-1% of the total, and
- Preservatives in a proportion of about 0,075-0,090% of the total.

2. An assembly according to claim 1, **characterized in that** it comprises disinfectants, antiseptics and antimicrobials in a proportion of about 0,2%-0,4% of the total.

## Patentansprüche

1. Kalte Applikatoranordnung, bei der ein schlauchförmiges Gewebe (1), das in eine flüssige Zusammensetzung eingetaucht ist, in einer luftdichten Verpackung enthalten ist, wobei die flüssige Zusammensetzung Folgendes umfasst:
- Wasser oder destilliertes Wasser, etwa 50-60 % der Gesamtmenge,
- denaturierten Alkohol, etwa 20-28 % der Gesamtmenge,
- Gefrierschutzmittel, etwa 15-20 % der Gesamtmenge,
- ein Detergens, etwa 1,5-2,5 % der Gesamtmenge,
- einen Vasodilatator 0,2-0,7 % der Gesamtmenge,
- Relaxanzien 0,7-1 % der Gesamtmenge, und
- Konservierungsmittel in einem Anteil von etwa 0,075-0,090 % der Gesamtmenge.

2. Anordnung nach Anspruch 1, **dadurch gekennzeichnet, dass** sie Desinfektionsmittel, Antiseptika und antimikrobielle Mittel in einem Anteil von etwa 0,2 % bis 0,4 % der Gesamtmenge enthält.

## Revendications

1. Ensemble applicateur de froid, dans lequel un tissu tubulaire (1) immergé dans une composition liquide est contenu dans un emballage étanche à l'air, dans lequel ladite composition liquide comprend :
- de l'eau ou de l'eau distillée, environ 50 à 60% du total,
- de l'alcool dénaturé, environ 20 à 28 % du total,
- un agent antigel, environ 15 à 20 % du total,
- un détergent, environ 1,5 à 2,5 % du total,
- un produit vasodilatateur, 0,2 à 0,7 % du total,
- des produits relaxants, 0,7 à 1 % du total, et
- des conservateurs en une proportion d'environ 0,075 à 0,090 % du total.

2. Ensemble selon la revendication 1, **caractérisé en ce qu'**il comprend des désinfectants, des antiseptiques et des antimicrobiens en une proportion d'environ 0,2 % à 0,4 % du total.
